(19) Europäisches Patentamt
European Patent Office
Office européen des brevets

(11) **EP 4 109 092 B1**

## (12) EUROPEAN PATENT SPECIFICATION

(45) Date of publication and mention
of the grant of the patent:
**25.10.2023  Bulletin 2023/43**

(21) Application number: **21181274.8**

(22) Date of filing: **23.06.2021**

(51) International Patent Classification (IPC):
**G01N 33/18** (2006.01)      **G01N 1/40** (2006.01)
**G01N 7/10** (2006.01)

(52) Cooperative Patent Classification (CPC):
**G01N 33/18; G01N 1/4005; G01N 7/10**

(54) **GAS-EQUILIBRIUM MEMBRANE INLET MASS SPECTROMETRY WITH ACCURATE QUANTIFICATION OF DISSOLVED-GAS PARTIAL PRESSURES**

GASGLEICHGEWICHTSMEMBRANEINLASS-MASSENSPEKTROMETRIE MIT GENAUER QUANTIFIZIERUNG DER PARTIALDRÜCKE GELÖSTER GASE

SPECTROMÉTRIE DE MASSE À ENTRÉE MEMBRANAIRE À L'ÉQUILIBRE GAZEUX AVEC QUANTIFICATION PRÉCISE DES PRESSIONS PARTIELLES DE GAZ DISSOUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**28.12.2022   Bulletin 2022/52**

(73) Proprietor: **Eidgenössische Anstalt für Wasserversorgung, Abwasserreinigung und Gewässerschutz, Eawag
8600 Dübendorf ZH (CH)**

(72) Inventors:
• **Brennwald, Matthias
8046 Zurich (CH)**
• **Kipfer, Rolf
8038 Zurich (CH)**

(74) Representative: **Kasche & Partner
Bühlstrasse 1
8125 Zollikerberg/Zürich (CH)**

(56) References cited:
• **BRENNWALD MATTHIAS S. ET AL: "A Portable and Autonomous Mass Spectrometric System for On-Site Environmental Gas Analysis", ENVIRONMENTAL SCIENCE & TECHNOLOGY, vol. 50, no. 24, 20 December 2016 (2016-12-20), pages 13455-13463, XP055873443, US ISSN: 0013-936X, DOI: 10.1021/acs.est.6b03669**
• **BRENNWALD MATTHIAS S. ET AL: "Deconvolution and compensation of mass spectrometric overlap interferences with the miniRUEDI portable mass spectrometer", METHODSX, vol. 7, 101038, 21 August 2020 (2020-08-21), pages 1-11, XP055873453, NL ISSN: 2215-0161, DOI: 10.1016/j.mex.2020.101038**

EP 4 109 092 B1

**Description**

The present invention is directed to a method for the quantification of dissolved gases in solvent solutions, optionally aqueous solutions, comprising the steps of (i) separating dissolved gases from a solvent solution, optionally an aqueous solution, by means of a gas permeable membrane at gas liquid equilibrium conditions, (ii) determining the total gas pressure $p_{tot}$ of the separated gases at the equilibrium conditions, (iii) determining the solvent partial pressure $p_{solv}$, optionally water partial pressure, (iv) feeding at least part of all separated gases to a gas analysis device, (v) determining the uncorrected partial pressures $x_i$ of all separated gases that significantly contribute to the total gas pressure $p_{tot}$ at the gas equilibrium conditions, and (vi) determining the sum of all uncorrected partial pressures $x_{tot}$, and (vii) determining the partial pressures $p_i$ of one or more dissolved gases. Furthermore, the present invention relates to a gas equilibrium-membrane inlet-based gas analysis device for the quantification of dissolved gases by the method described herein and corresponding uses of the method or the device for the quantification of partial pressures of dissolved gases in solvent, optionally aqueous solutions, optionally selected from the group consisting of drinking water, waste water, sewage water, natural waters, spring water, ocean water, lake water, biological fluids, urin, blood, or non-aqueous solutions, optionally selected from alcoholic solution, mineral and biological oils, gasoline, diesel, brake fluid, and hydraulic fluids.

Dissolved gases in solvent solutions such as aqueous and non-aqueous liquids are routinely analysed using gas analysis devices such as, e.g. mass spectrometers, gas chromatographs, alpha spectrometers, cavity-ring down spectrometers, or other optical analysers, etc. Some of these gas analysis devices employ capillary gas inlets and use a gas-permeable membrane for separating the solvent liquid from the gas inlet of the analysis device. One example is the so-called membrane-inlet mass spectrometry, MIMS. Gas-equilibrium membrane inlet mass spectrometry (GE-MIMS) is a special MIMS technique for the simple, quantitative analysis of the partial pressures of the gas species dissolved in the liquid (Brennwald et al., Environ. Sci. Technol. 2016, 50, 24, 13455-13463; Machler et al., Environ. Sci. Technol. 2012, 46, 15, 8288-8296; Cassar et al., Anal. Chem. 2009, 81 (5), 1855-1864; Manning et al., Anal. Chem. 2016, 88 (6), 3040-3048. Typically for the GE-MIMS technique, a solubility equilibrium is established between the sampled liquid and a gas headspace separated by a membrane. A small fraction of the equilibrated headspace gas flows through a capillary or other pressure reducing means into the vacuum system of a gas analysis device, where the gases are quantitatively analyzed.

In order to accurately calibrate the gas analysis device's readings in terms of the dissolved-gas partial pressures, the rate of gas flow from the headspace into the gas analyser must be known. However, the rate of the gas flow through the capillary is a complicated function which depends strongly and in a non-linear way on the total gas pressure at the capillary inlet, the viscosity of the gas (which in turn is determined by the temperature and composition of the gas), and further parameters (see G.M. Fryer: A theory of gas flow through capillary tubes. Proc. R. Soc. London, Ser. A, 1966, 293, 329-341).

In most practical applications, the named parameters are variable and a-priori unknown. Therefore, quantification of the gas flow through the capillary is notoriously difficult, which in turn makes accurate calibration of the gas-analysis readings challenging regarding the partial pressures of the dissolved gas.

As a result, present gas-equilibrium membrane inlet gas analysis devices, in particular gas-equilibrium membrane inlet mass spectrometry devices (GE-MIMS), and the corresponding analysis methods have the inherent problem that any change in parameters of the liquid sample to be analysed, e.g. temperature and/or composition changes, will necessitate one or many new calibrations of the device, in particular, if a continuous sampling with changing parameters is desired.

The calibration problem of gas-equilibrium membrane inlet gas analysis devices is solved in a first aspect by a method for the quantification of dissolved gases in solvent solutions comprising:

    (i) separating dissolved gases from a solvent solution by means of a gas permeable membrane at gas liquid equilibrium conditions,
    (ii) determining the total gas pressure $p_{tot}$ of the separated gases at the equilibrium conditions,
    (iii) determining the solvent partial pressure $p_{solv}$ in the separated gases, optionally from the temperature at the equilibrium conditions,
    (iv) feeding at least part of all separated gases to a gas analysis device, optionally by pressure-reducing means, optionally by capillary means,
    (v) determining the uncorrected partial pressures $x_i$ of all separated gases that significantly contribute to the total gas pressure $p_{tot}$ at the gas equilibrium conditions,
    (vi) determining the sum of all uncorrected partial pressures $x_{tot}$, and
    (vii) determining the partial pressures $p_i$ of one or more, optionally all dissolved gases.

The mixing ratio of the individual gases in the gas-equilibrium membrane inlet is not changed by the capillary pressure-reducing transport to the gas analysis device because the mostly viscous flow prevents a fractionation of the gases, i.e. the capillary transport is similar to a plug flow. The total gas pressure $p_{tot}$ in the gas sampling space at the membrane depends on the dissolution equilibrium of the gases to be analysed. The capillary gas flow rate depends on multiple and diverse parameters such as the capillary temperature, the capillary geometry, the gas composition, the gas viscosity and further parameters. The total gas pressure $p_{tot}$ in the membrane-based gas sampling space has unexpected utility as an accurate calibration factor for determining the partial pressures of the individual dissolved gases, as confirmed experimentally by the inventors. The total gas pressure $p_{tot}$ in the membrane-based gas sampling space can be continuously measured and used for continuous sampling and analysis procedures with gas-equilibrium membrane inlet gas analysis devices.

The method of the present invention has utility for any solvent solution capable of dissolving gas compounds and establishing a gas liquid equilibrium. The term solvent solution, as used herein, is meant to indicate a solution comprising a liquid solvent or solvent mixture capable of dissolving and having dissolved one or more gases capable of a gas liquid equilibrium state.

The method of the present invention has proven particularly useful for the quantification of dissolved gases in aqueous solutions and comprises the steps:

(i) separating dissolved gases from an aqueous solution by means of a gas permeable membrane at gas liquid equilibrium conditions,
(ii) determining the total gas pressure $p_{tot}$ of the separated gases at the equilibrium conditions,
(iii) determining the water partial pressure $p_{solv}$ in the separated gases, optionally from the temperature at the equilibrium conditions,
(iv) feeding at least part of all separated gases to a gas analysis device, optionally by pressure-reducing means, optionally by capillary means,
(v) determining the uncorrected partial pressures $x_i$ of all separated gases that significantly contribute to the total gas pressure $p_{tot}$ at the gas liquid equilibrium conditions,
(vi) determining the sum of all uncorrected partial pressures $x_{tot}$ and
(vii) determining the partial pressures $p_i$ of one or more, optionally all dissolved gases.

The term aqueous solution, as used herein, is meant to indicate the presence of water in the solution in an amount that is suitable to contributing to the dissolution of gases in the solution.

According to the present invention, the sum of all uncorrected partial pressures $x_{tot}$ in step (vi) is determined by formula (I):

$$x_{tot} = \sum_i x_i \ ,$$

and the partial pressures $p_i$ in step (vii) are determined by formula (II):

$$p_i = \frac{x_i}{x_{tot}} \times (p_{tot} - p_{Solv}).$$

The method of the present invention is, in principle, suitable for any gases that are capable of being dissolved in the corresponding solvent, optionally an aqueous solvent. For example, the dissolved gases for analysis according to the method of the present invention may be selected from the group consisting of He, Ne, Ar, Kr, Xe, Rn, $N_2$, $O_2$, $CO_2$, $CH_4$, $C_2H_6$, $C_3H_8$ and $H_2$.

The term equilibrium conditions, as used herein, is meant to indicate the physical and chemical parameters that control the gas/solvent partitioning (e.g. the solvent temperature, chemical composition, pH, salinity, etc.), at which solubility equilibrium is attained.

The gas liquid equilibrium conditions for separating dissolved gases from a solvent solution by a method of the present invention, optionally from an aqueous solution, require that the solvent remains liquid and non-boiling. For example, in step (i) and for aqueous solutions temperatures are optionally in the range of 0 to 90 °C, optionally 0 to 40 °C, optionally 5 to 35 °C and/or the pressure is atmospheric pressures.

In a further embodiment the gas analysis device for practicing the method of the present invention is a gas analysis device having a capillary-based gas inlet or similar unit controlling the gas flow into the analyser, optionally selected from the group consisting of a mass spectrometer, alpha spectrometers, optical spectrometers, cavity ring down spectrometers, and a gas chromatograph.

In a further aspect the present invention is directed to a gas equilibrium-membrane inlet-based analysis device for the quantification of dissolved gases in solvent solutions, optionally aqueous solutions, at equilibrium conditions by the method of the present invention, wherein the device comprises

(a) a gas permeable membrane for separating dissolved gases from a solvent solution, optionally an aqueous solution, at gas liquid equilibrium conditions, into a confined, gas impermeable, and constant volume container,
(b) a pressure sensor configured for determining the total gas pressure $p_{tot}$ of the separated gases at the equilibrium conditions,
(c) a temperature sensor configured for determining the temperature of the solvent at the equilibrium conditions,
(d) a gas analysis device configured for determining the uncorrected partial pressures $x_i$ of all separated gases that significantly contribute to the total gas pressure $p_{tot}$ in the gas analysis device, and
(e) a feed line for feeding at least part of all separated gases to the gas analysis device, optionally by pressure-reducing means, optionally by capillary means,
(f) one or more processors, and
(g) one or more memories storing software code portions executable by the one or more processors to enable performing the following:
(h) determining the solvent or optionally water saturation pressure $p_{solv}$ from the temperature deter-

mined by the temperature sensor (c),

(i) determining the sum of all uncorrected partial pressures $x_{tot}$ determined by the gas analysis device (d), and

(j) determining the partial pressures $p_i$ of one or more, optionally all of the gases separated from the solution in step (a).

For example, the gas permeable membrane for separating dissolved gases from a solvent solution of interest, optionally an aqueous solution, in feature (a) may be any membrane blocking the passage of the liquid solvent and providing for the passage of evaporating gases such that a solubility equilibrium of the gas/liquid solvent partitioning according to Henrys Law is established. Such membranes are state of the art for many gases and many solutions. Nonlimiting examples of membranes for use in the present invention are optionally made of hydrophobic porous polymers such as, for example, polypropylene (PP), polyethylene (PE), polytetrafluorethylene (PTFE), silicone, etc. and are available, for example, as "LiquiCel!", "Accurel".

The term confined, gas impermeable, and constant volume container gas container, as used herein, is meant to indicate any confinement of constant volume for the gas transferred through the gas-permeable membrane, e.g. a simple membrane headspace, from where the gases cannot escape or diffuse and are solely directed to the inlet of a gas analysis device with a capillary gas inlet.

The pressure sensor configured for determining the total gas pressure $p_{tot}$ of the separated gases at the equilibrium conditions in feature (b) can be positioned to reach into or contact the confined gas container or can otherwise be operably coupled with the container for determining the total gas pressure. The same, the temperature sensor configured for determining the temperature of the solvent at equilibrium conditions can be positioned to reach into and/or contact the liquid solvent solution for determining the temperature of the solvent solution at the equilibrium conditions.

The terms one or more processors and one or more memories storing software code portions executable by the one or more of the processors for executing features (h) to (j) for the device of the present invention are common general knowledge and are meant to be interpreted broadly as understood in the art of data processing.

For the device according to the present invention the sum of all uncorrected partial pressures $x_{tot}$ in step (i) is determined by formula (I):

$$x_{tot} = \sum_i x_i ,$$

and the partial pressures $p_i$ in step (j) are determined by formula (II):

$$p_i = \frac{x_i}{x_{tot}} \times (p_{tot} - p_{Solv}).$$

In one alternative, the device of the present invention is configured for the quantification of dissolved gases selected from the group consisting of He, Ne, Ar, Kr, Xe, Rn, $N_2$, $O_2$, $CO_2$, $CH_4$, $C_2H_6$, $C_3H_8$ and $H_2$.

In a further embodiment, the device of the present invention comprises a gas analysis device comprising a capillary-based gas inlet, that is optionally selected from the group consisting of a mass spectrometer, alpha spectrometers, optical spectrometers, cavity ring down spectrometers, and a gas chromatograph.

In another aspect the present invention encompasses the use of a method and/or a device of the present invention as described herein for the quantification of partial pressures of dissolved gases in solvent solution, optionally aqueous solutions.

In an optional embodiment, the use of the present invention pertains to the analysis of gases dissolved in solvent solutions, optionally aqueous solutions, optionally selected from the group consisting of drinking water, waste water, sewage water, natural waters, spring water, ocean water, lake water, biological fluids, urine, blood, or non-aqueous solutions, optionally selected from alcoholic solutions, mineral and biological oils, gasoline, diesel, brake fluid, and hydraulic fluids.

In the following the present invention is illustrated by means of figures and examples, none of which are to be interpreted as limiting the invention beyond the scope of the appended claims.

**Figures**

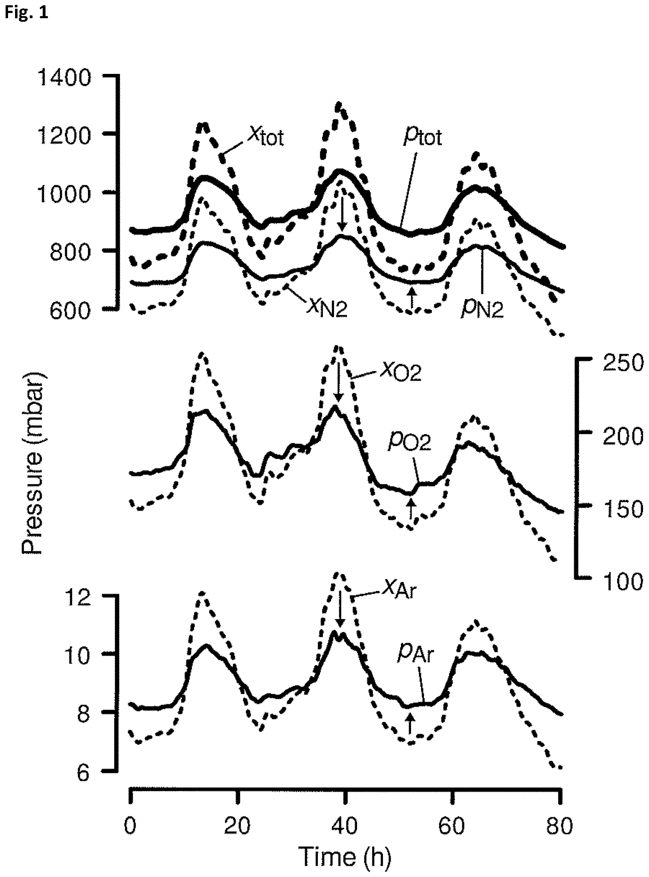

Fig. 1 shows a comparison of the analysis results obtained with the conventional GE-MIMS method ($x_i$, $x_{tot}$) and with the GE-MIMS-APP method of the present invention ($p_i$, $p_{tot}$) for the gases nitrogen (upper panel), oxygen (middle panel) and argon (lower panel) in tap water. Due to the non-linear response of the capillary-inlet of the gas analyzer, a mass spectrometry device, to the variation of the total gas pressure, the $x_i$ do not accurately reflect the true dissolved-gas partial pressures. This is confirmed by the amplitude of $x_{tot}$, which exceeds the amplitude of the true total gas pressure $p_{tot}$ as determined using the pressure sensor (upper panel). The corrected partial pressures $p_i$ determined using the GE-MIMS-APP method of the present invention exhibit a smaller amplitude than the corresponding $x_i$, which is consistent with the true total gas pressure.

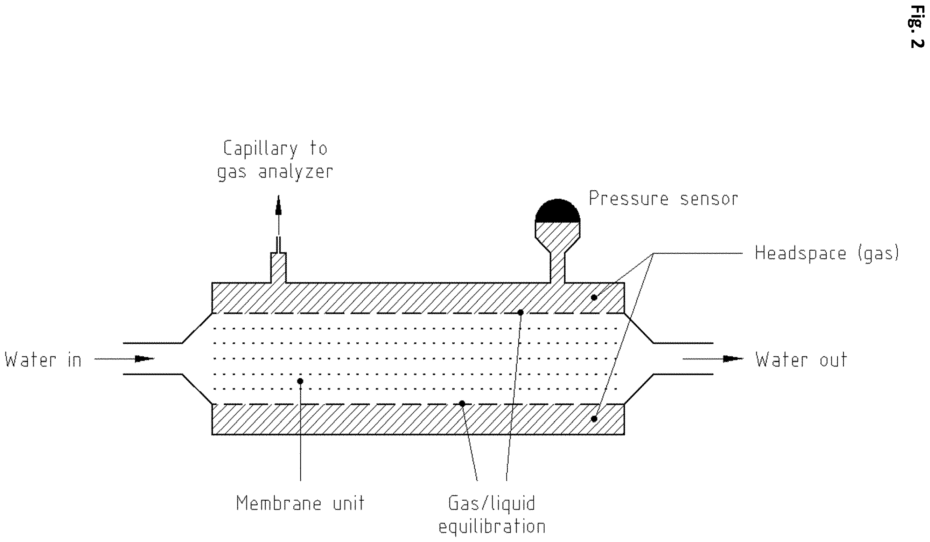

Fig. 2 is a simple schematic drawing of a gas-permeable membrane separation device for use with a gas analysis device (not shown), e.g. a mass spectrometry device, with a capillary pressure-reducing inlet, i.e. a capillary feed line, to the gas analysis device, and with a pressure sensor positioned in the membrane headspace for determining the total gas pressure $p_{tot}$ of the separated gases at equilibrium conditions. Also not shown is the temperature sensor configured for determining the temperature of the separated gases at the equilibrium conditions.

## Examples

The GE-MIMS-APP method of the present invention was demonstrated by analysing the partial pressures of the gases $N_2$, $O_2$ and Ar dissolved in tap water. The GE-MIMS analysis used a "miniRUEDI" gas analyzer (Brennwald et al., Environmental Science and Technology 50(24), DOI: 10.1021/acs.est.6b03669; a SRS RGA200 quadrupole mass spectrometer coupled to the gas headspace of a Liqui-Cel G542 membrane module (3M) using an 11m long stainless-steel capillary with 0.1mm internal diameter,). For GE-MIMS-APP, this setup was extended with a WIKA P30 pressure sensor coupled to the gas headspace of the membrane module. The water for analysis was filled in a tub (60 cm long, 40 cm wide, 15 cm deep) and exposed to ambient air. The water temperature increased during the day due to solar heating, and decreased due to cooling during the night. This diurnal temperature variation (18 - 37 °C) resulted in a corresponding variation of the gas solubilities, whereby the air/water gas exchange was too slow to maintain the aqueous concentrations of the gases at equilibrium relative to their atmospheric partial pressures. The partial pressures of the gases in the headspace of the GE-MIMS module were therefore in disequilibrium with the atmosphere, and showed a variation following the diurnal heating and cooling of the water.

## Claims

1. A method for the quantification of dissolved gases in solvent solutions comprising:

   (i) separating dissolved gases from a solvent solution by means of a gas permeable membrane at gas liquid equilibrium conditions,
   (ii) determining the total gas pressure $p_{tot}$ of the separated gases at the equilibrium conditions,
   (iii) determining the solvent partial pressure $p_{solv}$ in the separated gases, optionally from the solvent temperature at the equilibrium conditions,
   (iv) feeding at least part of all separated gases to a gas analysis device, optionally by pressure-reducing means, optionally by capillary means,
   (v) determining the uncorrected partial pressures $x_i$ of all separated gases that significantly contribute to the total gas pressure $p_{tot}$ at the gas liquid equilibrium conditions,
   (vi) determining the sum of all uncorrected partial pressures $x_{tot}$, and

(vii) determining the partial pressures $p_i$ of one or more, optionally all dissolved gases, wherein, in step (vi), the sum of all uncorrected partial pressures $x_{tot}$ is determined by formula (I):

$$x_{tot} = \sum_i x_i \, ,$$

and
the partial pressures $p_i$ step (vii) are determined by formula (II):

$$p_i = \frac{x_i}{x_{tot}} \times (p_{tot} - p_{Solv}).$$

2. The method of claim 1, wherein the solvent solutions are aqueous solutions.

3. The method of any of claims 1 to 2, wherein the dissolved gases are selected from the group consisting of He, Ne, Ar, Kr, Xe, Rn, $N_2$, $O_2$, $CO_2$, $CH_4$, $C_2H_6$, $C_3H_8$ and $H_2$.

4. The method of any of claims 1 to 3, wherein the gas liquid equilibrium conditions for separating dissolved gases from a solvent solution, optionally an aqueous solution in step (i) are in the range of 0 to 90, optionally 0 to 40 °C.

5. The method of any of claims 1 to 4, wherein the gas analysis device is a gas analysis device having a capillary-based gas inlet, optionally selected from the group consisting of a mass spectrometer, alpha spectrometers, optical spectrometers, cavity ring down spectrometers, and a gas chromatograph.

6. A gas equilibrium-membrane inlet-based analysis device for the quantification of dissolved gases in solvent solutions, optionally aqueous solutions at equilibrium conditions according to the method of any of claims 1 to 5, wherein the device comprises

   (a) a gas permeable membrane for separating dissolved gases from a solvent solution, optionally an aqueous solution, at gas liquid equilibrium conditions, into a confined, gas impermeable, and constant volume container,
   (b) a pressure sensor configured for determining the total gas pressure $p_{tot}$ of the separated gases at the equilibrium conditions,
   (c) a temperature sensor configured for determining the temperature of the solvent solution at the equilibrium conditions,
   (d) a gas analysis device configured for determining the uncorrected partial pressures $x_i$ of all separated gases that significantly contribute to the total gas pressure $p_{tot}$ in the gas analysis device, and

(e) a feed line for feeding at least part of all separated gases to the gas analysis device, optionally by pressure-reducing means, optionally by capillary means,

(f) one or more processors, and

(g) one or more memories storing software code portions executable by the one or more processors to enable performing the following:

(h) determining the solvent or optionally water partial pressure $p_{solv}$ in the separated gases from the temperature determined by the temperature sensor (c),

(i) determining the sum of all uncorrected partial pressures $x_{tot}$ determined by the gas analysis device (d), and

(j) determining the partial pressures $p_i$ of one or more, optionally all of the gases separated from the solution in step (a),

wherein the sum $x_{tot}$ of all uncorrected partial pressures $x_i$ in step (i) is determined by formula (I):

$$x_{tot} = \sum_i x_i \, ,$$

Z and Z
the partial pressures $p_i$ in step (j) are determined by formula (II):

$$p_i = \frac{x_i}{x_{tot}} \times (p_{tot} - p_{Solv}).$$

**7.** The device of claim 6, wherein the device is configured for the quantification of dissolved gases selected from the group consisting of He, Ne, Ar, Kr, Xe, Rn, $N_2$, $O_2$, $CO_2$, $CH_4$, $C_2H_6$, $C_3H_8$ and $H_2$.

**8.** The device of any of claims 6 or 7, wherein the gas analysis device comprises a capillary-based gas inlet, and is optionally selected from the group consisting of a mass spectrometer, alpha spectrometers, optical spectrometers, cavity ring down spectrometers, and a gas chromatograph.

**9.** Use of a method according to any of claims 1 to 5 or a device according to any of claims 6 to 8 for the quantification of partial pressures of dissolved gases in solvent solution, optionally aqueous solutions.

**10.** Use of a method or a device according to claim 9, wherein the solvent solutions are aqueous solutions, optionally selected from the group consisting of drinking water, waste water, sewage water, natural waters, spring water, ocean water, lake water, biological fluids, urine, blood, or non-aqueous solutions, optionally selected from alcoholic solutions, mineral and biological oils, gasoline, diesel, brake fluid, and hydraulic fluids.

## Patentansprüche

**1.** Ein Verfahren für die Quantifizierung von gelösten Gasen in lösungsmittelhaltigen Lösungen, das Folgendes umfasst:

(i) die Abtrennung gelöster Gase aus einer lösungsmittelhaltigen Lösung mittels einer gasdurchlässigen Membran bei Gas-Flüssigkeits-Gleichgewichtsbedingungen,

(ii) die Bestimmung des Gesamtgasdrucks $p_{tot}$ der abgetrennten Gase bei Gleichgewichtsbedingungen,

(iii) die Bestimmung des Lösungsmittelpartialdrucks $p_{solv}$ in den abgetrennten Gasen, optional aus der Lösungsmitteltemperatur bei den Gleichgewichtsbedingungen,

(iv) die Zuführung wenigstens eines Teils von allen abgetrennten Gasen zu einer Gasanalysenvorrichtung, optional durch druckreduzierende Mittel, optional durch Kapillarmittel,

(v) die Bestimmung der nicht korrigierten Teildrücke x;von allen abgetrennten Gasen, die wesentlich zu dem Gesamtgasdruck $p_{tot}$ bei den Gas-Flüssigkeits-Gleichgewichtsbedingungen beitragen,

(vi) die Bestimmung der Summe von allen nicht korrigierten Teildrücken $x_{tot}$, und

(vii) die Bestimmung der Teildrücke $p_i$ von einem oder mehreren, optional von allen gelösten Gasen,

wobei in Schritt (vi) die Summe von allen nicht korrigierten Teildrücken $x_{tot}$ durch Formel (I):

$$x_{tot} = \sum_i x_i \, ,$$

bestimmt wird, und
die Teildrücke $p_i$ in Schritt (vii) durch die Formel (II):

$$p_i = \frac{x_i}{x_{tot}} \times (p_{tot} - p_{Solv})$$

bestimmt werden.

**2.** Das Verfahren von Anspruch 1, wobei die lösungsmittelhaltigen Lösungen wässrige Lösungen sind.

**3.** Das Verfahren von einem der Ansprüche 1 bis 2, wobei die gelösten Gase aus der Gruppe ausgewählt sind, die aus He, Ne, Ar, Kr, Xe, Rn, $N_2$, $O_2$, $CO_2$, $CH_4$, $C_2H_6$, $C_3H_8$ und $H_2$ besteht.

**4.** Das Verfahren von einem der Ansprüche 1 bis 3, wobei die Gas-Flüssigkeits-Gleichgewichtsbedingungen für die Abtrennung von gelösten Gase aus einer lösungsmittelhaltigen Lösung, optional einer wässrigen Lösung in Schritt (i), in dem Bereich von 0 bis 90, optional 0 bis 40 °C liegen.

**5.** Das Verfahren von einem der Ansprüche 1 bis 4, wobei die Gasanalysenvorrichtung eine Gasanalysenvorrichtung mit einem kapillar-basierten Gaseinlass ist, und optional aus der Gruppe ausgewählt ist, die aus einem Massenspektrometer, Alpha-Spektrometern, optischen Spektrometern, Cavity-Ringdown-Spektrometern, und einem Gaschromatographen besteht.

**6.** Ein Analysenvorrichtung basierend auf einem Gas-Gleichgewichts-Membraneinlass für die Quantifizierung von gelösten Gasen in lösungsmittelhaltigen Lösungen, optional wässrigen Lösungen, bei Gleichgewichtsbedingungen gemäss dem Verfahren von einem der Ansprüche 1 bis 5, wobei die Vorrichtung Folgendes umfasst:

(a) eine gasdurchlässige Membran zur Abtrennung gelöster Gase aus einer lösungsmittelhaltigen Lösung, optional einer wässrigen Lösung, bei Gas-Flüssigkeits-Gleichgewichtsbedingungen, in einen geschlossenen, gasundurchlässigen Behälter mit konstantem Volumen,
(b) einen Drucksensor, der zur Bestimmung des Gesamtgasdrucks $p_{tot}$ der abgetrennten Gase bei den Gleichgewichtsbedingungen ausgelegt ist,
(c) einen Temperatursensor, der zur Bestimmung der Temperatur der lösungsmittelhaltigen Lösung bei Gleichgewichtsbedingungen ausgelegt ist,
(d) eine Gasanalysenvorrichtung, die zur Bestimmung der nicht korrigierten Teildrücke $x_i$ von allen abgetrennten Gasen ausgelegt ist, die wesentlich zu dem Gesamtgasdruck $p_{tot}$ in der Gasanalysenvorrichtung beitragen, und
(e) eine Zuleitung für das Zuführen wenigstens eines Teils von allen abgetrennten Gasen zu der Gasanalysenvorrichtung, optional durch druckreduzierende Mittel, optional durch Kapillarmittel,
(f) einen oder mehrere Prozessoren, und
(g) einen oder mehrere Datenspeicher, die Teile von Softwarecodes speichern, die durch den oder die mehreren Prozessoren ausgeführt werden, die die Durchführung des Folgenden ermöglichen:
(h) die Bestimmung des Teildrucks $p_{solv}$ des Lösungsmittels oder optional des Wassers in den abgetrennten Gasen aus der Temperatur, die durch den Temperatursensor (c) bestimmt wird,

(i) die Bestimmung der Summe von allen nicht korrigierten Teildrücken $x_{tot}$, die durch die Gasanalysenvorrichtung (d) bestimmt werden, und
(j) die Bestimmung der Teildrücke $p_i$ von einem oder mehreren, optional allen der aus der Lösung in Schritt (a) abgetrennten Gase.

wobei

die Summe $x_{tot}$ von allen nicht korrigierten Teildrücken $x_i$ in Schritt (i) durch Formel (I)

$$x_{tot} = \sum_i x_i$$

bestimmt wird, und
die Teildrücke $p_i$ in Schritt (j) durch Formel (II):

$$p_i = \frac{x_i}{x_{tot}} \times (p_{tot} - p_{Solv}).$$

bestimmt werden.

**7.** Die Vorrichtung von Anspruch 6, wobei die Vorrichtung für die Quantifizierung von gelösten Gasen, ausgewählt aus der Gruppe, die aus He, Ne, Ar, Kr, Xe, Rn, $N_2$, $O_2$, $CO_2$, $CH_4$, $C_2H_6$, $C_3H_g$ und $H_2$ besteht, ausgelegt ist.

**8.** Die Vorrichtung von einem der Ansprüche 6 oder 7, wobei die Gasanalysenvorrichtung einen kapillar-basierten Gaseinlass aufweist, und optional aus der Gruppe ausgewählt ist, die aus einem Massenspektrometer, Alpha-Spektrometern, optischen Spektrometern, Cavity-Ringdown-Spektrometern, und einem Gaschromatographen besteht.

**9.** Verwendung eines Verfahrens gemäss einem der Ansprüche 1 bis 5 oder einer Vorrichtung gemäss einem der Ansprüche 6 bis 8 für die Quantifizierung von Teildrücken von gelösten Gasen in lösungsmittelhaltigen Lösungen, optional in wässrigen Lösungen.

**10.** Verwendung eines Verfahrens oder einer Vorrichtung gemäss Anspruch 9, wobei die lösungsmittelhaltigen Lösungen wässrige Lösungen sind, optional ausgewählt aus der Gruppe bestehend aus Trinkwasser, Abwasser, Schmutzwasser, Naturwassern, Quellwasser, Meerwasser, Seewasser, biologischen Flüssigkeiten, Urin, Blut, oder nicht-wässrigen Lösungen, optional ausgewählt aus alkoholischen Lösungen, mineralischen und biologischen Ölen, Benzin, Bremsflüssigkeit und Hydraulikflüssigkeiten.

**Revendications**

1. Procédé pour la quantification de gaz dissous dans des solutions de solvant comprenant :

   (i) la séparation de gaz dissous d'une solution de solvant au moyen d'une membrane perméable aux gaz dans des conditions d'équilibre gaz-liquide ;
   (ii) la détermination de la pression de gaz totale $p_{tot}$ des gaz séparés dans lesconditions d'équilibre ;
   (iii) la détermination de la pression partielle de solvant $p_{solv}$ dans les gaz séparés, optionnellement à partir de la température de solvant dans les conditions d'équilibre ;
   (iv) l'alimentation en au moins une partie de tous les gaz séparés d'un dispositif d'analyse de gaz, optionnellement à l'aide d'un moyen de réduction de pression, optionnellement à l'aide d'un moyen capillaire ;
   (v) la détermination des pressions partielles non corrigées $x_i$ de tous les gaz séparés qui contribuent de façon significative à la pression de gaz totale $p_{tot}$ dans les conditions d'équilibre gaz-liquide ;
   (vi) la détermination de la somme de toutes les pressions partielles non corrigées $x_{tot}$ ; et
   (vii) la détermination des pressions partielles $p_i$ d'un ou de plusieurs gaz dissous, optionnellement de tous les gaz dissous, dans lequel, à l'étape (vi), la somme de toutes les pressions partielles non corrigées $x_{tot}$ est déterminée par la formule (I) :

   $$x_{tot} = \sum_i x_i \; ;$$

   et

   les pressions partielles $p_i$ à l'étape (vii) sont déterminées par la formule (II) :

   $$p_i = \frac{x_i}{x_{tot}} \times (p_{tot} - p_{solv}).$$

2. Procédé selon la revendication 1, dans lequel les solutions de solvant sont des solutions aqueuses.

3. Procédé selon l'une quelconque des revendications 1 à 2, dans lequel les gaz dissous sont sélectionnés parmi le groupe constitué par : He, Ne, Ar, Kr, Xe, Rn, $N_2$, $O_2$, $CO_2$, $CH_4$, $C_2H_6$, $C_3H_8$ et $H_2$.

4. Procédé selon l'une quelconque des revendications 1 à 3, dans lequel les conditions d'équilibre gaz-liquide pour séparer les gaz dissous d'une solution de solvant, optionnellement d'une solution aqueuse, à l'étape (i) s'inscrivent à l'intérieur de la plage de 0 à 90, optionnellement de 0 à 40 °C.

5. Procédé selon l'une quelconque des revendications 1 à 4, dans lequel le dispositif d'analyse de gaz est un dispositif d'analyse de gaz qui comporte une entrée de gaz capillaire, optionnellement sélectionné parmi le groupe constitué par un spectromètre de masse, des spectromètres alpha, des spectromètres optiques, des spectromètres à temps de déclin d'une cavité et un chromatographe en phase gazeuse.

6. Dispositif d'analyse à entrée membranaire à équilibre gazeux pour la quantification de gaz dissous dans des solutions de solvant, optionnellement des solutions aqueuses, à des conditions d'équilibre conformément au procédé selon l'une quelconque des revendications 1 à 5, dans lequel le dispositif comprend :

   (a) une membrane perméable aux gaz pour séparer des gaz dissous d'une solution de solvant, optionnellement une solution aqueuse, à des conditions d'équilibre gaz-liquide, à l'intérieur d'un conteneur confiné, imperméable aux gaz et de volume constant ;
   (b) un capteur de pression configuré pour déterminer la pression de gaz totale $p_{tot}$ des gaz séparés dans les conditions d'équilibre ;
   (c) un capteur de température configuré pour déterminer la température de la solution de solvant dans les conditions d'équilibre ;
   (d) un dispositif d'analyse de gaz configuré pour déterminer les pressions partielles non corrigées $x_i$ de tous les gaz séparés qui contribuent de façon significative à la pression de gaz totale $p_{tot}$ dans le dispositif d'analyse de gaz ; et
   (e) une ligne d'alimentation pour alimenter en au moins une partie de tous les gaz séparés le dispositif d'analyse de gaz, optionnellement à l'aide d'un moyen de réduction de la pression, optionnellement à l'aide d'un moyen capillaire ;
   (f) un ou plusieurs processeurs ; et
   (g) une ou plusieurs mémoires qui stockent des parties de code de logiciel pouvant être exécutées par les un ou plusieurs processeurs pour permettre la réalisation des étapes suivantes :
   (h) la détermination de la pression partielle du solvant, optionnellement de l'eau, $p_{solv}$ dans les gaz séparés à partir de la température déterminée par le capteur de température (c) ;
   (i) la détermination de la somme de toutes les pressions partielles non corrigées $x_{tot}$ déterminées par le dispositif d'analyse de gaz (d) ; et
   (j) la détermination des pressions partielles $p_i$ d'un ou de plusieurs gaz, optionnellement de tous les gaz, séparés de la solution à l'étape (a) ; dans lequel la somme $x_{tot}$ de toutes les pres-

sions partielles non corrigée $x_i$ à l'étape (i) est déterminée par la formule (I) :

$$x_{tot} = \sum_i x_i \;;$$

et

les pressions partielles $p_i$ à l'étape (j) sont déterminées par la formule (II) :

$$p_i = \frac{x_i}{x_{tot}} \times (p_{tot} - p_{solv}).$$

7. Dispositif selon la revendication 6, dans lequel le dispositif est configuré pour la quantification de gaz dissous sélectionnés parmi le groupe constitué par : He, Ne, Ar, Kr, Xe, Rn, $N_2$, $O_2$, $CO_2$, $CH_4$, $C_2H_6$, $C_3H_8$ et $H_2$.

8. Dispositif selon l'une quelconque des revendications 6 ou 7, dans lequel le dispositif d'analyse de gaz comprend une entrée de gaz capillaire et est optionnellement sélectionné parmi le groupe constitué par : un spectromètre de masse, des spectromètres alpha, des spectromètres optiques, des spectromètres à temps de déclin d'une cavité et un chromatographe en phase gazeuse.

9. Utilisation d'un procédé selon l'une quelconque des revendications 1 à 5 ou d'un dispositif selon l'une quelconque des revendications 6 à 8 pour la quantification de pressions partielles de gaz dissous dans une solution de solvant, optionnellement dans des solutions aqueuses.

10. Utilisation d'un procédé ou d'un dispositif selon la revendication 9, dans laquelle les solutions de solvant sont des solutions aqueuses, optionnellement sélectionnées parmi le groupe constitué par : l'eau potable, les eaux usées, les eaux d'égout, les eaux naturelles, l'eau de source, l'eau de mer, l'eau de lac, les fluides biologiques, l'urine, le sang, ou les solutions non aqueuses, optionnellement sélectionnées parmi les solutions alcooliques, les huiles minérales et biologiques, l'essence, le diesel, le liquide de frein et les fluides hydrauliques.

**Fig. 1**

**Fig. 2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **BRENNWALD et al.** *Environ. Sci. Technol.,* 2016, vol. 50 (24), 13455-13463
- **MACHLER et al.** *Environ. Sci. Technol.,* 2012, vol. 46 (15), 8288-8296
- **CASSAR et al.** *Anal. Chem.,* 2009, vol. 81 (5), 1855-1864
- **MANNING et al.** *Anal. Chem.,* 2016, vol. 88 (6), 3040-3048
- **G.M. FRYER.** A theory of gas flow through capillary tubes. *Proc. R. Soc. London, Ser. A,* 1966, vol. 293, 329-341
- **BRENNWALD et al.** *Environmental Science and Technology,* vol. 50 (24